# EUROPEAN PATENT SPECIFICATION

(11) **EP 3 257 461 B1**
(45) Date of publication and mention of the grant of the patent: **22.05.2019**
(21) Application number: 17175428.6
(22) Date of filing: 12.06.2017
(51) Int. Cl.: A61B 18/12, A61B 18/00

(54) **VARIABLE ACTIVE SNUBBER CIRCUIT TO INDUCE ZERO-VOLTAGE-SWITCHING IN A CURRENT-FED POWER CONVERTER**
VARIABLE AKTIVE DÄMPFERSCHALTUNG ZUM INDUZIEREN VON NULL-SPANNUNGSSCHALTUNG IN EINEM STROMGESPEISTEN LEISTUNGSWANDLER
CIRCUIT D'AMORTISSEMENT ACTIF VARIABLE POUR INDUIRE UNE COMMUTATION À TENSION NULLE DANS UN CONVERTISSEUR DE PUISSANCE ALIMENTÉ PAR COURANT

(30) Priority: 13.06.2016 US 201615180180
(43) Date of publication of application: 20.12.2017
(73) Proprietor: Covidien LP, Mansfield, MA 02048 (US)
(72) Inventor: SMITH, Robert B., Loveland, CO Colorado 80538 (US); RUPP, Steven C., Arvada, CO Colorado 80004 (US); FRIEDRICHS, Daniel A., Aurora, CO Colorado 80012 (US)
(74) Representative: Maschio, Antonio

(56) References cited:
- EP-A1- 2 491 881
- EP-A2- 2 826 433
- US-A1- 2014 276 754

## Description

### BACKGROUND

### Technical Field

The present disclosure relates to systems and methods for controlling an electrosurgical generator. In particular, the present disclosure relates to a current source electrosurgical generator having a power converter and a variable active snubber circuit configured to achieve zero voltage switching within the power converter.

### Background of Related Art

Electrosurgery involves application of high radio frequency electrical current to a surgical site to cut, ablate, desiccate, or coagulate tissue. In monopolar electrosurgery, a source or active electrode delivers radio frequency alternating current from the electrosurgical generator to the targeted tissue. A patient return electrode is placed remotely from the active electrode to conduct the current back to the generator.

In bipolar electrosurgery, return and active electrodes are placed in close proximity to each other such that an electrical circuit is formed between the two electrodes (e.g., in the case of an electrosurgical forceps). In this manner, the applied electrical current is limited to the body tissue positioned between the electrodes. Accordingly, bipolar electrosurgery generally involves the use of instruments where it is desired to achieve a focused delivery of electrosurgical energy between two electrodes. EP2826433 discloses an electrosurgical generator according to the preamble of claim 1.

Conventional electrosurgical generators may utilize voltage-fed or current-fed power converters. Current-fed power converters have a number of advantages over voltage-fed converters including control of arcs, desirable transient performance, and simplified control dynamics. However, current-fed power converters also present a number of problems, such as power dissipation, which limits their usability. Accordingly, there is a need for a system and method to control an electrosurgical generator including a current-fed power converter that overcomes these problems.

### SUMMARY

The invention is defined by the electrosurgical generator of independent claim 1 and by the method of independent claim 6. Preferred embodiments are defined by the dependent claims. The present disclosure provides for an electrosurgical generator including a power converter having a plurality of switching elements, such as FETs. The generator also includes a current source, which may be an inductor, and a variable active snubber circuit. The current source supplies current to the power converter, whereas the variable active snubber circuit mitigates some of the effects of the current source on the power converter to achieve zero voltage switching therein.

Electrosurgical generators according to the present disclosure may include a current source having a relatively large inductance to smooth the current supplied to the power converter. In particular, the inductance of the current source provides advantages over voltage-fed generators, as it naturally regulates and stabilizes the current to downstream elements, e.g., power converter. This allows for better control of arcing, e.g., generating or mitigating arcing, and minimizing transient performance.

Electrosurgical generators including voltage-fed converters operate based on switching on/off of switching-elements to control the power. In contrast, electrosurgical generators including current-fed converters control power delivery by shorting the current to ground, or some other return path. Thus, when the voltage-fed converter turns on all of the switching elements, this results in large power dissipation with destructive currents. To deal which these surges, at least one of the switching elements remains open or off at all times. For the current-fed converter, shorting or shunting the current source, e.g., an inductor, no significant power is dissipated while the current flow is maintained. The problem for the current-fed converters, however, is that if all of the switching elements are off at the same time, such that the current flow is interrupted, destructive voltages are generated, as the current flowing in the inductor is interrupted. Conversely, when the switching elements are turned on in the current-fed converter, the full normal load voltage is present across the switching elements. This results in significant power dissipation in the switching elements, large noise, voltage and current spikes, and potentially large radiated and conducted electromagnetic interference. In particular, sensitive circuits, e.g., sense circuits, may be impacted and measurement and control may be degraded and other equipment in the operating room may also be disrupted due to these surges. This is highly undesirable because switching losses can become significant enough to cause excessive heating of various components and require larger, more robust components and cooling.

The switching elements of the power converter operate as voltage-controlled resistive devices and due to their construction do not dissipate heat efficiently. Thus, even when the switching elements are turned off, large currents are passing through them even as the voltage across the switching elements remains very close to zero for the nanoseconds required for the switching element to actually stop conducting. However, when the switching element turns on, any voltage across the device is shunted very quickly as the switching element begins to conduct. This results in the large switching losses associated with current-fed generators as described above.

The generator according to the present disclosure provides for a variable active snubber circuit which induces zero-voltage switching in the power converter to deal with the above-described switching losses associated with current-fed power converters. Bringing the voltage across the switching elements to zero prior to turning them on, eliminates the large current spikes associated with the load voltage. Accordingly, this also overcomes the above-described disadvantages of using the current source in an electrosurgical generator. Previous attempts to deal with this problem involved the use of auxiliary circuits to provide shunting of the current by secondary elements. These configurations involve some form of pre-shunting of the current to reduce or eliminate the voltage before the switching elements are closed. Although these circuits mitigate the problem, they are not entirely effective, as they introduce additional current and noise spikes, albeit of diminished magnitude.

According to one embodiment of the present disclosure, an electrosurgical generator is provided. The electrosurgical generator includes: a power supply configured to output a direct current; a current source coupled to the power supply and configured to output source current based on the direct current; and a power converter coupled to the current source, the power converter including at least one power switching element operated at a switching waveform. The power converter is configured to generate a converted waveform based on the source current. The electrosurgical generator also includes a controller coupled to the power converter and configured to modulate the switching waveform and a snubber circuit coupled to the current source and the power converter. The snubber circuit is configured to return the voltage at the at least one power switching element to zero after the power converter generates at least a portion of the converted waveform.

According to another embodiment of the present disclosure, an electrosurgical generator is provided. The electrosurgical generator includes: a power supply configured to output direct current; a current source coupled to the power supply and configured to output source current based on the direct current; and a power converter coupled to the current source, the power converter including four power switching elements arranged in an H-bridge topology and operated at a switching waveform. The power converter is configured to generate a converted waveform based on the source current. The electrosurgical generator also includes a controller coupled to the power converter and configured to modulate the switching waveform and a snubber circuit coupled to the current source and the power converter. The snubber circuit is configured to return the voltage at each of the power switching elements to zero after the power converter generates at least a portion of the converted waveform.

According to an aspect of any of the above embodiments, the snubber circuit may include a snubber inductor, a snubber capacitor, and a snubber catch diode, all of which are interconnected in series. The snubber circuit may further include at least one snubber switching element coupling the snubber inductor with the snubber capacitor and the snubber catch diode. Voltage at the snubber inductor rises and is clamped by the snubber catch diode and charges the snubber capacitor in response to deactivation of the power converter. Current in the snubber inductor is reversed after the snubber capacitor is charged and the current from the snubber inductor causes ring-back to counteract capacitance of the power converter.

According to another aspect of any of the above embodiments, the controller is coupled to the snubber circuit and is configured to control the at least one snubber switching element to maintain a desired voltage in the snubber capacitor.

According to a further embodiment of the present disclosure a method for controlling an electrosurgical generator is provided. The method includes: activating a first pair of power switching elements and a second pair of power switching elements of a power converter; increasing current at a current source coupled to the power converter; deactivating the first pair of the power switching elements to generate a radio frequency pulse; deactivating at least one power switching element of the second pair of the power switching elements; and activating a snubber circuit coupled to the current source and the power converter to return the voltage at each of the power switching elements to zero prior to reactivating the first pair of power switching elements and the second pair of power switching elements.

According to an aspect of the above embodiment, activating the snubber circuit may include increasing voltage at a snubber inductor of the snubber circuit.

According to another aspect of the above embodiment, activating the snubber circuit may further include clamping the current at the snubber inductor by a snubber catch diode of the snubber circuit in response to deactivating the at least one power switching element of the second pair of the power switching elements.

According to a further aspect of the above embodiment, activating the snubber circuit may further include charging a snubber capacitor of the snubber circuit.

According to yet another aspect of the above embodiment, activating the snubber circuit may further include controlling the at least one snubber switching element of the snubber circuit to maintain a desired voltage in the snubber capacitor.

### BRIEF DESCRIPTION OF THE DRAWINGS

The present disclosure may be understood by reference to the accompanying drawings, when considered in conjunction with the subsequent, detailed description, in which:
Fig. 1 is a perspective view of a surgical system according to an embodiment of the present disclosure;
Fig. 2 is a front view of an electrosurgical generator of Fig. 1 according to an embodiment of the present disclosure;
Fig. 3 is a schematic diagram of the electrosurgical generator of Fig. 2 according to an embodiment of the present disclosure;
Fig. 4 is a schematic diagram of a variable active snubber circuit of the electrosurgical generator of Fig. 2 according to the present disclosure;
Fig. 5 is a plot of a switching waveform, a generator output waveform, a power converter waveform, and a current source waveform according to the present disclosure; and
Fig. 6 is a flow chart of a method for operating the electrosurgical generator of Fig. 2 according to an embodiment of the present disclosure.

### DETAILED DESCRIPTION

Particular embodiments of the present disclosure will be described below with reference to the accompanying drawings. In the following description, well-known functions or constructions are not described in detail to avoid obscuring the present disclosure in unnecessary detail. Those skilled in the art will understand that the present disclosure may be adapted for use with either an endoscopic instrument, a laparoscopic instrument, or an open instrument. It should also be appreciated that different electrical and mechanical connections and other considerations may apply to each particular type of instrument.

A generator may be used in monopolar and/or bipolar electrosurgical procedures, including, for example, cutting, coagulation, ablation, and vessel sealing procedures. The generator may include a plurality of outputs for interfacing with various ultrasonic and electrosurgical instruments (e.g., ultrasonic dissectors and hemostats, monopolar instruments, return electrode pads, bipolar electrosurgical forceps, footswitches, etc.). Further, the generator may include electronic circuitry configured to generate radio frequency energy specifically suited for powering ultrasonic instruments and electrosurgical devices operating in various electrosurgical modes (e.g., cut, blend, coagulate, division with hemostasis, fulgurate, spray, etc.) and procedures (e.g., monopolar, bipolar, vessel sealing).

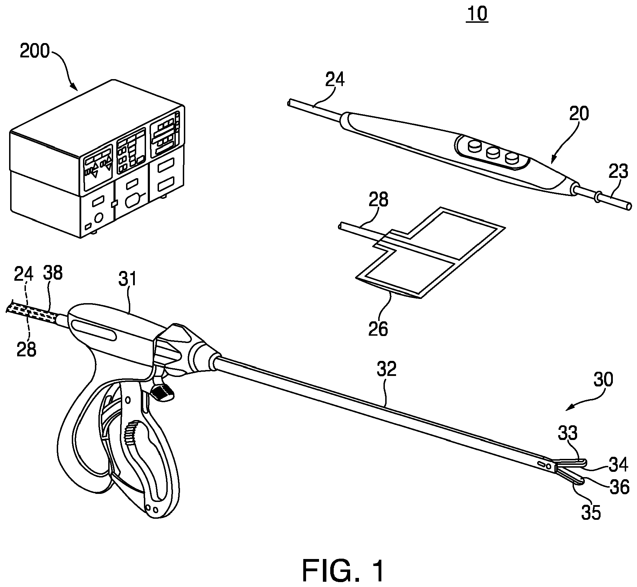

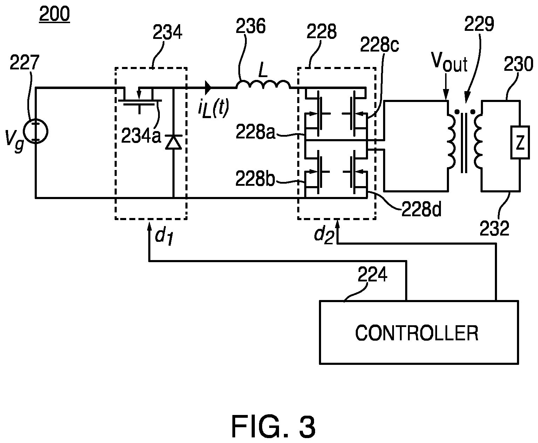

Fig. 1 is a perspective view of the components of one illustrative embodiment of an electrosurgical system 10 according to the present disclosure. The system 10 may include one or more monopolar electrosurgical instruments 20 having one or more active electrodes 23 (e.g., electrosurgical cutting probe, ablation electrode(s), etc.) for treating tissue of a patient. Electrosurgical alternating RF current is supplied to the instrument 20 by a generator 200 via a supply line 24 that is connected to an active terminal 230 (Fig. 3) of the generator 200, allowing the instrument 20 to cut, coagulate, thermally or non-thermally ablate and/or otherwise treat tissue. The alternating current is returned to the generator 200 through a return electrode pad 26 via a return line 28 at a return terminal 232 (Fig. 3) of the generator 200. For monopolar operation, the system 10 may include a plurality of return electrode pads 26 that, in use, are disposed on a patient to minimize the chances of tissue damage by maximizing the overall contact area with the patient. In addition, the generator 200 and the return electrode pads 26 may be configured for monitoring tissue-to-patient contact to ensure that sufficient contact exists therebetween.

The system 10 may also include one or more bipolar electrosurgical instruments, for example, a bipolar electrosurgical forceps 30 having one or more electrodes for treating tissue of a patient. The electrosurgical forceps 30 includes a housing 31 and opposing jaw members 33 and 35 disposed at a distal end of a shaft 32. The jaw members 33 and 35 have one or more active electrodes 34 and a return electrode 36 disposed therein, respectively. The active electrode 34 and the return electrode 36 are connected to the generator 200 through cable 38 that includes the supply and return lines 24, 28, which may be coupled to the active and return terminals 230, 232, respectively (Fig. 3). The electrosurgical forceps 30 is coupled to the generator 200 at a port having connections to the active and return terminals 230 and 232 (e.g., pins) via a plug disposed at the end of the cable 38, wherein the plug includes contacts from the supply and return lines 24, 28 as described in more detail below.

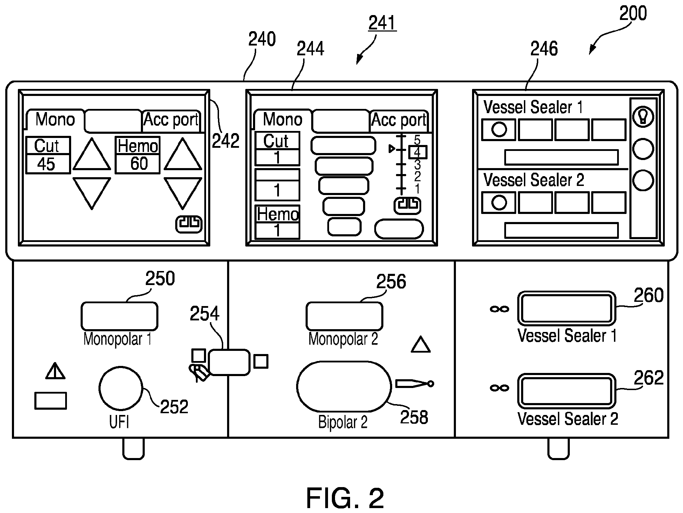

With reference to Fig. 2, a front face 240 of the generator 200 is shown. The generator 200 may include a plurality of ports 250-262 to accommodate various types of electrosurgical instruments (e.g., monopolar electrosurgical instrument 20, electrosurgical forceps 30, etc.).

The generator 200 includes a user interface 241 having one or more display screens 242, 244, 246 for providing the user with variety of output information (e.g., intensity settings, treatment complete indicators, etc.). Each of the screens 242, 244, 246 is associated with a corresponding port 250-262. The generator 200 includes suitable input controls (e.g., buttons, activators, switches, touch screen, etc.) for controlling the generator 200. The screens 242, 244, 246 are also configured as touch screens that display a corresponding menu for the instruments (e.g., electrosurgical forceps 30, etc.). The user then adjusts inputs by simply touching corresponding menu options.

Screen 242 controls monopolar output and the devices connected to the ports 250 and 252. Port 250 is configured to couple to a monopolar electrosurgical instrument (e.g., electrosurgical instrument 20) and port 252 is configured to couple to a foot switch (not shown). The foot switch provides for additional inputs (e.g., replicating inputs of the generator 200). Screen 244 controls monopolar and bipolar output and the devices connected to the ports 256 and 258. Port 256 is configured to couple to other monopolar instruments. Port 258 is configured to couple to a bipolar instrument (not shown).

Screen 246 controls the electrosurgical forceps 30 that may be plugged into one of the ports 260 and 262, respectively. The generator 200 outputs energy through the ports 260 and 262 suitable for sealing tissue grasped by the electrosurgical forceps 30. In particular, screen 246 outputs a user interface that allows the user to input a user-defined intensity setting for each of the ports 260 and 262. The user-defined setting may be any setting that allows the user to adjust one or more energy delivery parameters, such as power, current, voltage, energy, etc. or sealing parameters, such as energy rate limiters, sealing duration, etc. The user-defined setting is transmitted to a controller 224 (Fig. 3) where the setting may be saved in memory. In embodiments, the intensity setting may be a number scale, such as for example, from one to ten or one to five. In embodiments, the intensity setting may be associated with an output curve of the generator 200. The intensity settings may be specific for each electrosurgical forceps 30 being utilized, such that various instruments provide the user with a specific intensity scale corresponding to the electrosurgical forceps 30. The active and return terminals 230 and 232 may be coupled to any of the desired ports 250-262. In embodiments, the active and return terminals 230 and 232 may be coupled to the ports 250-262.

Fig. 3 shows a schematic block diagram of the generator 200, which includes a controller 224, a power supply 227, and a power converter 228. The power supply 227 may be a high voltage, DC power supply connected to an AC source (e.g., line voltage) and provides high voltage, DC power to the power converter 228, which then converts high voltage, DC power into RF energy and delivers the energy to the active terminal 230. The energy is returned thereto via the return terminal 232. In particular, electrosurgical energy for energizing the monopolar electrosurgical instrument 20 and/or electrosurgical forceps 30 is delivered through the active and return terminals 230 and 232. The active and return terminals 230 and 232 may be coupled to the power converter 228 through an isolation transformer 229. The isolation transformer 229 is optional and the active and return terminals 230 and 232 may be coupled directly to converter 228.

The generator 200 also includes a DC-DC buck converter 234 coupled to the power supply 227. Furthermore, a current source 236 is electrically coupled to the DC-DC buck converter 234 and the power converter 228. The current source 236 may be an inductor having an inductance which smoothes the current supplied to the power converter 228. The current source 236 is configured to supply current to the power converter 228. The output of power converter 228 transmits current through an isolation transformer 229 to the load "Z", e.g., tissue being treated.

The power converter 228 is configured to operate in a plurality of modes, during which the generator 200 outputs corresponding waveforms having specific duty cycles, peak voltages, crest factors, etc. It is envisioned that in other embodiments, the generator 200 may be based on other types of suitable power supply topologies. Power converter 228 may be a resonant RF amplifier or a non-resonant RF amplifier. A non-resonant RF amplifier, as used herein, denotes an amplifier lacking any tuning components, e.g., conductors, capacitors, etc., disposed between the power converter and the load "Z."

The controller 224 includes a processor (not shown) operably connected to a memory (not shown), which may include one or more of volatile, non-volatile, magnetic, optical, or electrical media, such as read-only memory (ROM), random access memory (RAM), electrically-erasable programmable ROM (EEPROM), non-volatile RAM (NVRAM), or flash memory. The processor may be any suitable processor (e.g., control circuit) adapted to perform the operations, calculations, and/or set of instructions described in the present disclosure including, but not limited to, a hardware processor, a field programmable gate array (FPGA), a digital signal processor (DSP), a central processing unit (CPU), a microprocessor, and combinations thereof. Those skilled in the art will appreciate that the processor may be substituted for by using any logic processor (e.g., control circuit) adapted to perform the calculations and/or set of instructions described herein.

The controller 224 includes an output port that is operably connected to the power supply 227 and/or power converter 228 allowing the processor to control the output of the generator 200 according to either open and/or closed control loop schemes. A closed loop control scheme is a feedback control loop, in which a plurality of sensors measure a variety of tissue and energy properties (e.g., tissue impedance, tissue temperature, output power, current and/or voltage, etc.), and provide feedback to the controller 224. The controller 224 then controls the power supply 227 and/or power converter 228, which adjusts the DC and/or power supply, respectively.

The generator 200 according to the present disclosure may also include a plurality of sensors (not shown). The sensors may be coupled to the power supply 227, the current source 234, and/or power converter 228 and may be configured to sense properties of DC current supplied to the power converter 228 and/or RF energy outputted by the power converter 228, respectively. Various components of the generator 200, namely, the power converter 228, the current and voltage sensors, may be disposed on a printed circuit board (PCB). The controller 224 also receives input signals from the input controls of the generator 200, the instrument 20 and/or electrosurgical forceps 30. The controller 224 utilizes the input signals to adjust power outputted by the generator 200 and/or performs other control functions thereon.

The DC-DC buck converter 234 includes a switching element 234a and power converter 228 includes a plurality of switching elements 228a-228d arranged in an H-bridge topology. In embodiments, power converter 228 may be configured according to any suitable topology including, but not limited to, half-bridge, full-bridge, push-pull, and the like. Suitable switching elements include voltage-controlled devices such as transistors, field-effect transistors (FETs), combinations thereof, and the like.

The controller 224 is in communication with both DC-DC buck converter 234 and power converter 228, in particular, the switching elements 234a and 228a-228d, respectively. Controller 224 is configured to output control signals, which may be a pulse-width modulated signal, to switching elements 234a and 228a-228d as described in further detail in co-pending application published as US 2014/0254221, filed on December 4, 2013 by Johnson et al.. In particular, controller 224 is configured to modulate a control signal d₁ supplied to switching element 234a of DC-DC buck converter 234 and control signals d₂ supplied to switching elements 228a-228d of power converter 228. Additionally, controller 224 is configured to measure power characteristics of generator 200, and control generator 200 based at least in part on the measured power characteristics. Examples of the measured power characteristics include the current through inductor 103 and the voltage at the output of power converter 228.

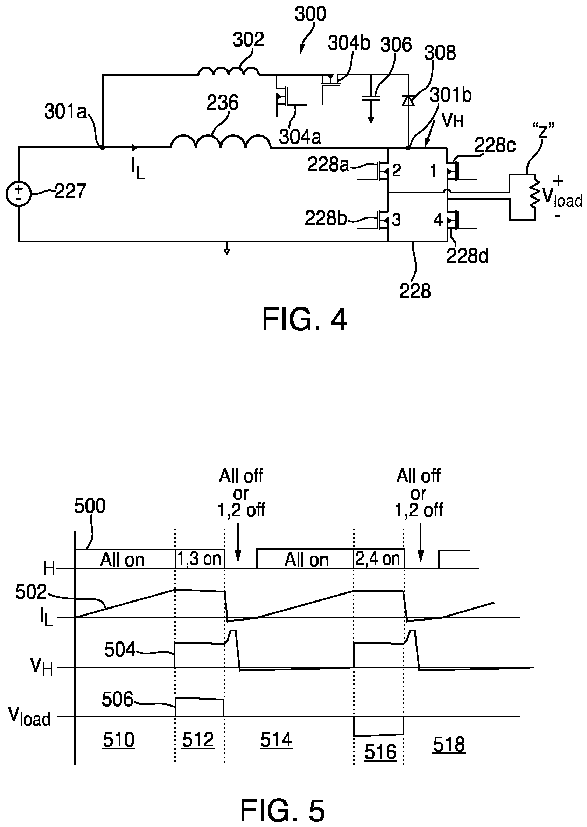

With reference to Fig. 4, the power converter 228 of the generator 200 is shown as a current-source power converter, which achieves zero-voltage-switching using a variable active snubber circuit 300. The snubber circuit 300 is coupled in parallel with the current source 236. The snubber circuit 300 includes a first node 301a disposed between the power supply 227 and the current source 236 and a second node 301b coupled to the power converter 228. The snubber circuit 300 includes a snubber inductor 302, a first switching element 304a, and a second switching element 304b. The first switching element 304a is connected to a ground and the second switching element 304b is connected in series with the power converter 228. The snubber circuit 300 also includes a snubber capacitor 306 and a snubber catch diode 308. The first and second switching elements 304a and 304b are configured to be switched at a fixed duty cycle by the controller 204 to establish the desired voltage at the snubber capacitor 306. The voltage in the snubber capacitor 306 is constantly being controlled via the first and second switching elements 304a and 304b and the snubber inductor 302 to maintain the desired capacitor voltage, which produces the desired ring-back at the second node 301b as described in more detail below.

Although Fig. 4 does not show the DC-DC buck converter 234, the snubber circuit 300 may be implemented with the DC-DC buck converter 234. In which case, the first node 301a would return to the DC-DC buck converter 234 rather than the power supply 227 as shown in Fig. 4.

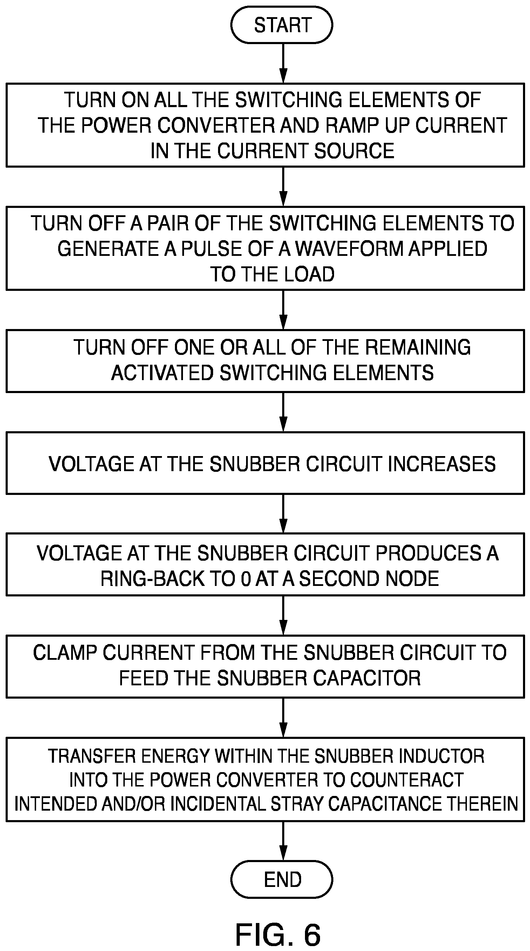

Fig. 5 illustrates a plurality of waveforms, namely, a switching waveform 500 for switching the switching elements 228a-228d, a waveform 502 generated by the current source 236, a converted waveform 504 generated by the power converter 228, and an RF waveform 506 at the load "Z." A method for operating the generator 200, and in particular the snubber circuit 300, is shown as a flow chart 600 in Fig. 6 and is described below with reference to Fig. 5.

Initially, during period 510 as shown in Fig. 5, all of the switching elements are turned on by the switching waveform 500 and current in the current source 236 ramps up to a desired predetermined level. Once the desired current is achieved, which may be determined by sensors (not shown) coupled to the controller 224, or by timing of the ramp time, one pair of the switching elements 228a-228d, e.g., switching element 228a and 228d or 228b and 228c, are turned off. This generates a first RF pulse (e.g., positive half cycles) that is supplied to the load "Z" during period 512.

At a predetermined time, during period 514, one of the high side switching elements, namely, switching element 228a or 228b, of the pair of the switching elements 228a and 228d or 228b and 228c, is also tuned off. As a result, all but one of the switching elements 228a-228d is turned off, namely, one of the low side switching elements 228c or 228d remains on. In embodiments, all of the remaining activated switching elements may also be turned off. In response to turning off the switching elements 228a-228d, the voltage at the output of the snubber inductor 302 rises very rapidly and is clamped by the snubber catch diode 308, which then feeds the energy into the snubber capacitor 306. In further embodiments, the power supply 227 feeding the current source 236 may also be turned off depending on specific requirements of downstream elements.

Due to the higher voltage at the snubber inductor 302, the energy in the snubber inductor 302 quickly dissipates and the current in the snubber inductor 302 reverses, causing ring-back due to the intended and incidental stray capacitance of the H-bridge circuitry of the power converter 208. In particular, the current from the snubber inductor 302 of the snubber 300 counteracts stray capacitance of the power converter 208. As the ring-back occurs, the voltage at the power converter 208 returns to zero.

During period 516, all of the switching element 228a-228d turn on to start the next cycle, which results in a reverse RF pulse (e.g., the negative half of the waveform 506 as compared to the pulse generated previously during period 512). The application of switching waveform 500 is repeated indefinitely to generate the desired waveform 506. In this implementation all forward or downstream current paths are turned off at the same time. As described above, this would be a catastrophic state for a conventional current-fed power supply were it not for the snubber circuit 300 according to the present disclosure.

While several embodiments of the disclosure have been shown in the drawings and/or described herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope of the claims appended hereto.

## Claims

1. An electrosurgical generator, comprising:
a power supply configured to output direct current;
a current source coupled to the power supply and configured to output a source current based on the direct current;
a power converter coupled to the current source, the power converter including at least one power switching element operated at a switching waveform, the power converter configured to generate a converted waveform based on the source current;
a controller coupled to the power converter and configured to modulate the switching waveform; and
a snubber circuit coupled to the current source and the power converter, the snubber circuit configured to return a voltage at the at least one power switching element to zero after the power converter generates at least a portion of the converted waveform **characterised in that** the snubber circuit includes a snubber inductor, a snubber capacitor, and a snubber catch diode, all of which are interconnected in series.

2. The electrosurgical generator according to claim 1, wherein the power converter includes four power switching elements arranged in an H-bridge topology.

3. The electrosurgical generator according to claim 1 or 2, wherein the snubber circuit further includes at least one snubber switching element coupling the snubber inductor to the snubber capacitor and the snubber catch diode.

4. The electrosurgical generator according to claim 3, wherein the snubber catch diode is configured to clamp a voltage at the snubber inductor and to charge the snubber capacitor in response to deactivation of the power converter, preferably wherein the snubber inductor is configured to reverse a current therethrough after the snubber capacitor is charged to generate ring-back to counteract capacitance of the power converter.

5. The electrosurgical generator according to claim 3 or 4, wherein the controller is coupled to the snubber circuit and is configured to control the at least one snubber switching element to maintain a desired voltage in the snubber capacitor.

6. A non-surgical method for controlling an electrosurgical generator, the method comprising:
activating a first pair of power switching elements and a second pair of power switching elements of a power converter;
increasing current at a current source coupled to the power converter;
deactivating the first pair of the power switching elements to generate a radio frequency pulse;
deactivating at least one power switching element of the second pair of the power switching elements; and
activating a snubber circuit coupled to the current source and the power converter to return voltage at each of the power switching elements to zero prior to reactivating the first pair of power switching elements and the second pair of power switching elements, **characterised in that** the snubber circuit includes a snubber inductor, a snubber capacitor, and a snubber catch diode, all of which are interconnected in series.

7. The method according to claim 6, wherein activating the snubber circuit includes:
increasing voltage at the snubber inductor of the snubber circuit.

8. The method according to claim 7, wherein activating the snubber circuit further includes clamping current at the snubber inductor by the snubber catch diode of the snubber circuit in response to deactivating the at least one power switching element of the second pair of the power switching elements.

9. The method according to claim 8, wherein activating the snubber circuit further includes charging the snubber capacitor of the snubber circuit.

10. The method according to claim 9, wherein activating the snubber circuit further includes controlling at least one snubber switching element of the snubber circuit to maintain a desired voltage in the snubber capacitor.

## Patentansprüche

1. Elektrochirurgischer Generator, umfassend:
eine Stromversorgung, die konfiguriert ist, um Gleichstrom auszugeben;
eine Stromquelle, die mit der Stromversorgung gekoppelt und konfiguriert ist, um einen Quellenstrom basierend auf dem Gleichstrom auszugeben;
einen Stromwandler, der mit der Stromquelle gekoppelt ist, wobei der Stromwandler mindestens ein Stromschaltelement aufweist, das mit einer Schaltwellenform betrieben wird, wobei der Stromwandler konfiguriert ist, um eine umgewandelte Wellenform basierend auf dem Quellenstrom zu erzeugen;
eine Steuerung, die mit dem Stromwandler gekoppelt und konfiguriert ist, um die Schaltwellenform zu modulieren; und
eine Snubber-Schaltung, die mit der Stromquelle und dem Stromwandler gekoppelt ist, wobei die Snubber-Schaltung konfiguriert ist, um eine Spannung an dem mindestens einen Stromschaltelement auf Null zurückzusetzen, nachdem der Stromwandler mindestens einen Teil der umgewandelten Wellenform erzeugt hat, **dadurch gekennzeichnet, dass** die Snubber-Schaltung eine Snubber-Spule, einen Snubber-Kondensator und eine Snubber-Freilaufdiode aufweist, die alle in Reihe geschaltet sind.

2. Elektrochirurgischer Generator nach Anspruch 1, wobei der Stromwandler vier Stromschaltelemente aufweist, die in einer H-Brücken-Topologie angeordnet sind.

3. Elektrochirurgischer Generator nach Anspruch 1 oder 2, wobei die Snubber-Schaltung weiter mindestens ein Snubber-Schaltelement aufweist, das die Snubber-Spule mit dem Snubber-Kondensator und der Snubber-Freilaufdiode koppelt.

4. Elektrochirurgischer Generator nach Anspruch 3, wobei die Snubber-Freilaufdiode konfiguriert ist, um eine Spannung an der Snubber-Spule zu klemmen und den Snubber-Kondensator als Reaktion auf eine Deaktivierung des Stromwandlers aufzuladen; vorzugsweise wobei die Snubber-Spule konfiguriert ist, um einen Strom durch diese hindurch umzukehren, nachdem der Snubber-Kondensator aufgeladen wurde, um ein Rückschwingen zu erzeugen, um einer Kapazität des Stromwandlers entgegenzuwirken.

5. Elektrochirurgischer Generator nach Anspruch 3 oder 4, wobei die Steuerung mit der Snubber-Schaltung gekoppelt und konfiguriert ist, um mindestens ein Snubber-Schaltelement zu steuern, um eine gewünschte Spannung in dem Snubber-Kondensator aufrechtzuerhalten.

6. Nicht-chirurgisches Verfahren zum Steuern eines elektrochirurgischen Generators, wobei das Verfahren umfasst:
Aktivieren eines ersten Paares von Stromschaltelementen und eines zweiten Paares von Stromschaltelementen eines Stromwandlers;
Erhöhen eines Stromes an einer mit dem Stromwandler gekoppelten Stromquelle;
Deaktivieren des ersten Paares der Stromschaltelemente, um einen Hochfrequenzpuls zu erzeugen;
Deaktivieren mindestens eines Stromschaltelements des zweiten Paares der Stromschaltelemente; und
Aktivieren einer Snubber-Schaltung, die mit der Stromquelle und dem Stromwandler gekoppelt ist, um eine Spannung an jedem der Stromschaltelemente auf Null zurückzusetzen, vor einem Reaktivieren des ersten Paares von Stromschaltelementen und des zweiten Paares von Stromschaltelementen, **dadurch gekennzeichnet, dass** die Snubber-Schaltung eine Snubber-Spule, einen Snubber-Kondensator und eine Snubber-Freilaufdiode aufweist, die alle in Reihe geschaltet sind.

7. Verfahren nach Anspruch 6, wobei Aktivieren der Snubber-Schaltung aufweist:
Erhöhen einer Spannung an der Snubber-Spule der Snubber-Schaltung.

8. Verfahren nach Anspruch 7, wobei Aktivieren der Snubber-Schaltung weiter aufweist Klemmen des Stroms an der Snubber-Spule durch die Snubber-Freilaufdiode der Snubber-Schaltung als Reaktion auf ein Deaktivieren des mindestens einen Stromschaltelements des zweiten Paares der Stromschaltelemente.

9. Verfahren nach Anspruch 8, wobei Aktivieren der Snubber-Schaltung weiter Aufladen des Snubber-Kondensators der Snubber-Schaltung aufweist.

10. Verfahren nach Anspruch 9, wobei Aktivieren der Snubber-Schaltung weiter Steuern mindestens eines Snubber-Schaltelements der Snubber-Schaltung, um eine gewünschte Spannung in dem Snubber-Kondensator aufrechtzuerhalten, aufweist.

## Revendications

1. Générateur électrochirurgical comprenant :
une alimentation électrique configurée pour délivrer en sortie un courant continu,
une source de courant couplée à l'alimentation électrique et configurée pour délivrer en sortie un courant de source sur la base du courant continu ;
un convertisseur de puissance couplé à la source de courant, le convertisseur de puissance incluant au moins un élément de commutation de puissance activé à une forme d'onde de commutation, le convertisseur de puissance étant configuré pour générer une forme d'onde convertie sur la base du courant de source ;
un dispositif de commande couplé au convertisseur de puissance et configuré pour moduler la forme d'onde de commutation ; et
un circuit d'amortissement couplé à la source de courant et au convertisseur de puissance, le circuit d'amortissement étant configuré pour ramener une tension dans le au moins un élément de commutation de puissance à zéro une fois que le convertisseur de puissance a généré au moins une partie de la forme d'onde convertie, **caractérisé en ce que** le circuit d'amortissement comprend un inducteur d'amortissement, un condensateur d'amortissement et une diode de blocage d'amortissement, qui sont tous interconnectés en série.

2. Générateur électrochirurgical selon la revendication 1, dans lequel le convertisseur de puissance inclut quatre éléments de commutation de puissance agencés selon une topologie à pont H.

3. Générateur électrochirurgical selon la revendication 1 ou 2, dans lequel le circuit d'amortissement inclut en outre au moins un élément de commutation d'amortissement couplant l'inducteur d'amortissement au condensateur d'amortissement et à la diode de blocage d'amortissement.

4. Générateur électrochirurgical selon la revendication 3, dans lequel la diode de blocage d'amortissement est configurée pour bloquer une tension dans l'inducteur d'amortissement et charger le condensateur d'amortissement en réponse à la désactivation du convertisseur de puissance, de préférence dans lequel l'inducteur d'amortissement est configuré pour inverser un courant qui le traverse une fois que le condensateur d'amortissement a été chargé pour générer un rappel afin de contrebalancer la capacité du convertisseur de puissance.

5. Générateur électrochirurgical selon la revendication 3 ou 4, dans lequel le dispositif de commande est couplé au circuit d'amortissement et est configuré pour commander le au moins un élément de commutation d'amortissement afin de maintenir une tension souhaitée dans le condensateur d'amortissement.

6. Procédé non chirurgical pour commander un générateur électrochirurgical, le procédé comprenant les étapes consistant à :
activer une première paire d'éléments de commutation de puissance et une seconde paire d'éléments de commutation de puissance d'un convertisseur de puissance,
augmenter le courant dans une source de courant couplée au convertisseur de puissance ;
désactiver la première paire d'éléments de commutation de puissance pour générer une impulsion de fréquence radio ;
désactiver au moins un élément de commutation de puissance de la seconde paire d'éléments de commutation de puissance ; et
activer un circuit d'amortissement couplé à la source de courant et au convertisseur de puissance pour ramener la tension dans chacun des éléments de commutation de puissance à zéro avec de réactiver la première paire d'éléments de commutation de puissance et la seconde paire d'élément de commutation de puissance, **caractérisé en ce que** le circuit d'amortissement inclut un inducteur d'amortissement, un condensateur d'amortissement et une diode de blocage d'amortissement, qui sont tous interconnectés en série.

7. Procédé selon la revendication 6, dans lequel l'activation du circuit d'amortissement inclut :
l'augmentation de la tension dans l'inducteur d'amortissement du circuit d'amortissement.

8. Procédé selon la revendication 7, dans lequel l'activation du circuit d'amortissement comprend en outre un courant de blocage dans l'inducteur d'amortissement par la diode de blocage d'amortissement du circuit d'amortissement en réponse à la désactivation du au moins un élément de commutation de puissance de la seconde paire d'éléments de commutation de puissance.

9. Procédé selon la revendication 8, dans lequel l'activation du circuit d'amortissement inclut en outre le chargement du condensateur d'amortissement du circuit d'amortissement.

10. Procédé selon la revendication 9, dans lequel l'activation du circuit d'amortissement inclut en outre la commande d'au moins un élément de commutation d'amortissement du circuit d'amortissement pour maintenir une tension souhaitée dans le condensateur d'amortissement.
